# EUROPEAN PATENT SPECIFICATION

(11) **EP 2 531 187 B1**
(45) Date of publication and mention of the grant of the patent: **12.08.2015**
(21) Application number: 11701683.2
(22) Date of filing: 03.02.2011
(51) Int. Cl.: A61K 31/18, A61K 31/194, A61K 31/427, A61K 31/4418, A61K 31/498, A61K 31/4985, A61K 31/506, A61K 31/519, A61K 31/53, A61K 45/06, A61P 11/00

(54) **sGC STIMULATORS OR sGC ACTIVATORS ALONE AND IN COMBINATION WITH PDE5 INHBITORS FOR THE TREATMENT OF CYSTIC FIBROSIS**
SGC-STIMULATOREN ODER SGC-AKTIVATOREN ALLEIN UND IN KOMBINATION MIT PDE5-HEMMERN ZUR BEHANDLUNG VON ZYSTISCHER FIBROSE
STIMULATEURS DE SGC OU ACTIVATEURS DE SGC SEULS OU EN COMBINAISON AVEC DES INHIBITEURS DE PDE5 POUR LE TRAITEMENT DE LA MUCOVISCIDOSE

(30) Priority: 05.02.2010 EP 10152727
(43) Date of publication of application: 12.12.2012
(73) Proprietor: Adverio Pharma GmbH, 12529 Schönefeld (DE)
(72) Inventor: SANDNER, Peter, 42113 Wuppertal (DE); VON DEGENFELD, Georges, 51373 Leverkusen (DE); STASCH, Johannes-Peter, 42651 Solingen (DE)
(86) International application number: PCT/EP2011/051532
(87) International publication number: WO 2011/095534

(56) References cited:
- WO-A1-2009/149278
- WO-A2-2007/039155
- DE-A1-102005 016 345
- US-A1- 2004 087 591
- US-A1- 2008 081 816
- CHEN LAN ET AL: "Mechanisms of cystic fibrosis transmembrane conductance regulator activation by S-nitrosoglutathione.", THE JOURNAL OF BIOLOGICAL CHEMISTRY 7 APR 2006 LNKD- PUBMED:16421103, vol. 281, no. 14, 7 April 2006 (2006-04-07), pages 9190-9199, XP002630667, ISSN: 0021-9258
- EVGENOV OLEG V ET AL: "NO-independent stimulators and activators of soluble guanylate cyclase: discovery and therapeutic potential", NATURE REVIEWS. DRUG DISCOVERY, NATURE PUBLISHING GROUP, GB, vol. 5, no. 9, 1 September 2006 (2006-09-01), pages 755-768, XP002534424, ISSN: 1474-1776, DOI: DOI:10.1038/NRD2038 cited in the application
- CLARKE LANE L: "Phosphodiesterase 5 inhibitors and cystic fibrosis: correcting chloride channel dysfunction.", AMERICAN JOURNAL OF RESPIRATORY AND CRITICAL CARE MEDICINE 1 MAR 2008 LNKD- PUBMED:18296466, vol. 177, no. 5, 1 March 2008 (2008-03-01) , pages 469-470, XP002630668, ISSN: 1535-4970 cited in the application
- LUBAMBA BOB ET AL: "Preclinical evidence that sildenafil and vardenafil activate chloride transport in cystic fibrosis.", AMERICAN JOURNAL OF RESPIRATORY AND CRITICAL CARE MEDICINE 1 MAR 2008 LNKD- PUBMED:18006891, vol. 177, no. 5, 1 March 2008 (2008-03-01) , pages 506-515, XP002630669, ISSN: 1535-4970 cited in the application
- DORMER R L ET AL: "Sildenafil (Viagra) corrects deltaF508-CFTR location in nasal epithelial cells from patients with cystic fibrosis", THORAX, BMJ PUBLISHING GROUP, GB, vol. 60, no. 1, 1 January 2005 (2005-01-01), pages 55-59, XP008125970, ISSN: 0040-6376, DOI: DOI:10.1136/THX.2003.019778 cited in the application
- SIM JI-YEON: "Nitric oxide and pulmonary hypertension.", KOREAN JOURNAL OF ANESTHESIOLOGY JAN 2010, vol. 58, no. 1, January 2010 (2010-01), pages 4-14, ISSN: 2005-7563
- GBEKOR EUGENE ET AL: "Phosphodiesterase 5 inhibitor profiles against all human phosphodiesterase families: Implications for use as pharmacological tools", JOURNAL OF UROLOGY, vol. 167, no. 4 Supplement, April 2002 (2002-04), page 246, & ANNUAL MEETING OF THE AMERICAN UROLOGY ASSOCIATION, INC.; ORLANDO, FLORIDA, USA; MAY 25-30, 2002 ISSN: 0022-5347

## Description

The present invention relates to the use of sGC stimulators and sGC activators as stand alone treatment, or in combination with PDE5 inhibitors, for preparation of medicaments for the treatment of Cystic Fibrosis (CF).

### Background of the invention

The cyclic nucleotides, cyclic adenosine monophosphate (cAMP) and cyclic guanosine monophosphate (cGMP), were discovered decades ago and represent one of the most important second messenger pathway within cells. It is well established that the regulation of intra-cellular cGMP pools have substantial impact on physiology, and pathophysiology and is one basic principle of pharmacological intervention (Eugenov et al. 2006; Schmidt et al. 2009). Nitrates and PDE5 inhibitors (PDE5i) which could increase intra-cellular cGMP levels are therefore already approved therapies for Angina, and Pulmonary Arterial Hypertension (PAH) or Erectile Dysfunction (ED), respectively. More recently discovered sGC stimulators and activators, are in advanced states of clinical development for PAH and Heart Failure. Therefore, targeting the NO/cGMP pathway by either cGMP production (nitrates, sGC stimulators, sGC activators) or cGMP break-down (PDE5i) became a very effective pharmacological intervention strategy in various diseases.

On a molecular level, NO-production results in stimulation of the soluble guanylate cyclase (sGC) resulting in enhanced cGMP formation. Consecutively, cGMP regulates different downstream targets, mainly cGMP regulated protein kinases (G-Kinases), cGMP-regulated phosphodiesterases (PDEs) and cGMP regulated ion channels which translates the NO-signal and rise in cGMP and in a decrease of intracellular free calcium. Therefore, the most prominent response of increasing intracellular cGMP, especially in the Smooth Muscle Cell (SMC), is relaxation. In addition, antiproliferative, antifibrotic or proapoptotic effects of cGMP are discussed and might expand the treatment options for PDE5 inhibitors (Sandner et al. 2007, Schmidt et al. 2009). More recently some lines of evidence showed that PDE5 inhibitors could also influence chloride secretion via the chloride channel CFTR and might be useful for the treatment of Cystic Fibrosis (Clarke 2008).

Cystic fibrosis (CF) is one of the most prevalent genetic disorders, caused by mutation of a single gene, the CFTR-channel, affecting 1 out of 2500-3000 newborns. In this disease, abnormal ion transport across the respiratory epithelia leads to dehydrated airway surface and viscous and poorly-cleared mucus. This contributes to chronic infections of the airways and high morbidity and early mortality. Up to now, the treatment is mainly focused on anti-infective treatment and lung transplantation but no causal therapy focusing on the correction and potentiation of impaired CFTR function is available.

On the molecular level a mutation in the CFTR gene results in CF. A broad variety of CF-causing mutations in the CFTR gene have been identified. However, the most prevalent mutation is a deletion of the phenylalanine in position 508 of the CFTR amino acid sequence, and is termed as ΔF508-CFTR. This mutation occurs in approximately 70%-80% of the cases of CF and is associated with a severe disease.

The deletion of residue 508 in ΔF508-CFTR prevents the mature protein from correct processing and folding. This missfolded CFTR could not, or not completely, exit the ER, and traffic to the plasma membrane. As a result, the number of channels present in the membrane in CFTR-patients is far less than observed in cells expressing wild-type CFTR. In addition the mutated channel exhibited reduced channel activity. Both, the reduced number of channels in the epithelial membranes and the reduced channel activity results in significantly impaired anion transport across epithelia leading to defective ion and fluid transport. This causes an imbalance in lung epithelial fluid transport and finally an excessive accumulation of viscosous mucus in the lungs. Moreover impaired CFTR-function also influences e.g. pancreatic function, gastro-intestinal functions, liver function, functions of secretory glands or insulin secretion.

In summary, impaired CFTR function by several mutations cause cystic fibrosis. Therefore correction and/or potentiation of CFTR function of these mutations could present a causal treatment option for Cystic Fibrosis (CF).

In addition correction and/or potentiation of CFTR function could present a causal treatment option for pancreatic dysfunction, liver dysfunction, dry mouth, dry eye, Sojegren's syndrome, and CF-induced diabetes.

It was shown, that in lung epithelial cells sildenafil - a potent and selective PDE5i - increased CFTR-driven chloride secretion (Cobb et. al 2003). In addition trafficking and functional activity of mutated CFTR-channels to the cell membrane could be influenced by PDE5i in vitro (Dormer et. al 2005, Carlile et al. 2007, Robert et al. 2008). In line with these findings it was demonstrated that PDE5 inhibitors when used in animal models of Cystic fibrosis (CF) are able to reduce mucin secretin (Mc Pherson 1999) and could influence chloride secretion (Lubamba et al. 2008). Therefore it was hypothesized that PDE5 inhibitors could be used for the treatment of Cystic Fibrosis (Cobb 1999, Lubamba et. al. 2008, Clarke 2008).

However, the use of PDE5 inhibitors is limited since they could only inhibit cGMP degradation. In cases in which NO-dependent cGMP production is low, their efficacy is at least partially impaired. Very interestingly, compounds have been described recently that could overcome this limitation of PDE5 inhibitors via direct stimulation or activation of the sGC.

In CHEN LAN ET AL: "Mechanisms of cystic fibrosis transmembrane conductance regulator activation by S-nitrosoglutathione.",THE JOURNAL OF BIOLOGICAL CHEMISTRY 7 APR 2006 LNKDPUBMED:16421 103, vol. 281, no. 14, 7April2006 (2006-04-07), pages 9190-91 99 it is shown in Calu3 cells that S-nitrosoglutathione may cGMP-dependently modulate CFTR-function - as measured by Chen et al. in *in vitro* using chamber experiments - this have only limited impact for CF patients. CF patients have mutated CFTR-channels, i.e. deIF508 CFTR is one of the most prevalent mutations among them and correction of WT-CFTR as expressed by Calu3 cells is not predictive for correction of mutated, i.e. deltaF508 CFTR function. Therefore, conclusions drawn from these experiments do not apply for CFTR function in Cystic fibrosis patients. This was the reasons why cell lines transfected with mutant CFTR channels were used for characterization of compounds of this application.

WO 2009/1 49278 AI (SYNERGY PHARMACEUTICALS INC [US]; SHAILUBHAI KUNWAR [US]; JACOB GARY S)10 December 2009 (2009-12-10) discloses peptidic gunaylin and urogyanilin analoges which target the membrane bound guanylate cyclase (GC-C). GC-C is predominantly expressed in intestinal epithelial cells and serves as the receptor for the gastrointestinal hormones guanylin and uroguanylin. Thus, conclusions for effects of these peptides, but especially for sGC stimulators and sGC activators, targeting the soluble guanylate cyclase which is a different receptor inside the cell could not be drawn from these data.

Two classes of compounds have been identified that activate the sGC NO-independently, the heme-dependent sGC stimulators, such as BAY 41-2272 according to compound of the formula (4a), BAY 41-8543 according to compound of the formula (1), and BAY 63-2521 according to compound of the formula (3), and heme-independent sGC activators, such as BAY 58-2667 according to compound of the formula (5), and HMR-1766 according to compound of the formula (6), (Stasch et al. 2001, for review see Evgenov et al. 2006).

We therefore investigated sGC stimulators and sGC activators, i.e. BAY 41-2272, BAY 60-4552 according to compound of the formula (4a, 4), alone or in combinations with PDE5 inhibitors, i.e. vardenafil, in vitro in cells and also in human tissue samples and in vivo, in CFTR-transgenic animal models.

In particular, PDE5 inhibitors, sGC stimulators and sGC activators alone or combinations thereof, were tested:
- in BHK-cells expressing a mutant CFTR channel, i.e. a tagged deltaF508 CFTR channel, on cell surface expression of the delta F508 CFTR.
- in HEK-293 cells expressing a mutant CFTR channel, i.e. a deltaF508CFTR, on CFTR function via measurement of iodine influx through fluoresence.
- in HEK-293 cells expressing a mutant CFTR channel, i.e. a deltaF508CFTR, on enhanced biosynthesis (band B) and processing (band C).
- in CFBE410⁻ cells expressing a mutant CFTR channel, i.e. a delta F508CFTR, on transepithelial conductance in ussing chamber experiments.
- in rectal biopsies of CF-patients and compared with rectal biopsies of non-CF patients on transepithelial conductance in using chamber experiments.
- in transgenic mice expressing the delta F508CFTR channel, on nasal potential difference and salivation.
- in transgenic mice not expressing the CFTR, on nasal potential difference and salivation.

We could demonstrate for the first time, that stimulators and activators of the soluble guanylate cyclase, corrected and potentiated CFTR function, presenting a new causal treatment option for CF-patients. In addition, we found completely unexpected that combinations of sGC stimulators or sGC activators with PDE5i showed more than additive effects on correction and potentiation. Therefore combinations might present a first causal and effective therapy for the treatment of Cystic Fibrosis (CF).

In particular, we found that PDE5 inhibitors could correct and potentiate CFTR function, i.e. delta F508 CFTR function in vitro. In addition, we found that sGC stimulators and activators - which have not been used so far - could correct and potentiate CFTR function, i.e. delta F508CFTR function. Finally we discovered that combinations of sGC stimulators or sGC activators with PDE5 inhibitors are superior to monotherapy and showed over-additive effects on CFTR function, i.e. delta F508 CFTR function.

In summary we discovered that combinations of sGC stimulators or sGC activators alone and in combination with PDE5 inhibitors could correct and potentiale CFTR function in vitro and in vivo. Due to this efficacy of sGC stimulators or sGC activators and combinations of sGC stimulators or sGC activators with PDE5 inhibitors, these stand alone and combination treatment could be used for the treatment of Cystic Fibrosis (CF). Pulmonary disorders, addressed by therapeutic agents of the invention which in particular and with substantial advantage can be treated by the above mentioned sGC stimulators or sGC activators alone, or in combination with PDE5 inhibitors, are lung diseases, comprising Cystic Fibrosis (CF)

Secretory disorders, addressed by therapeutic agents of the invention which in particular and with substantial advantage can be treated by the above mentioned sGC stimulators or sGC activators alone, or in combination with PDE5 inhibitors, comprising but not limited to pancreatetic dysfunction, gastrointestinal dysfunction, liver diseases, and cystic-fibrosis related diabetes mellitus (CFRD).

Other diseases, addressed by therapeutic agents of the invention which in particular and with substantial advantage can be treated by the above mentioned sGC stimulators or sGC activators alone, or in combination with PDE5 inhibitors, including, but not limited to, chronic obstructive pulmonary disease (COPD), dry eye disease, dry mouth diseases, and Sjoegren's Syndrome.

All diseases, addressed by therapeutic agents of the invention which in particular and with substantial advantage can be treated by the above mentioned sGC stimulators or sGC activators alone, or in combination with PDE5 inhibitors, are disease in which altered chloride secretion via CFTR is causally involved.

### Disclosure of the invention

The invention provides sGC stimulators or sGC activators alone, or in combination whith PDE5 inhibitors which are useful for the treatment of Cystic Fibrosis (CF), and superior in efficacy over methods of treatment already known.

The invention provides sGC stimulators or sGC activators alone, or in combination whith PDE5 inhibitors which are useful for the treatment of Cystic Fibrosis (CF), and superior in the side effect profile over methods of treatment already known.

Guanylate cyclase (sGC) stimulator and sGC activator is preferably a compound selected from the group consisting of
- 2-[1-(2-fluorobenzyl)-1H-pyrazolo[3,4-b]pyridine-3-yl]-5-(4-morpholinyl)-4,6-pyrimidinediamine (1), described also as example 16 in WO 00/06569,
- 2-[1-(2-fluorobenzyl)-1H-pyrazolo[3,4-b]pyridine-3-yl]-5-(4-pyridinyl)-4-pyrimidine-amine (2), described also as example 1 in WO 02/42301,
- methyl-4,6-diamino-2-[1-(2-fluorobenzyl)-1H-pyrazolo[3,4-b]pyridine-3-yl]-5-pyrimidinyl(methyl)carbamate (3), described also as example 8 in WO 03/095451,
- methyl-4,6-diamino-2-[1-(2-fluorobenzyl)-1H-pyrazolo[3,4-b]pyridin-3-yl]-5-pyrimidinylcarbamate (4), described also as example 5 in WO 03/095451,
- 3-(4-amino-5-cyclopropylpyrimidine-2-yl)-1-(2-fluorobenzyl)1H-pyrazolo[3,4-b]pyridine (4a), described also as example 1 in WO 00/06568,
and
- 4-({(4-carboxybutyl)[2-(2-{[4-(2-phenylethyl)benzyl]oxy}phenyl)ethyl]amino}methyl) benzoic acid (5), described also as example 8a in WO 01/019780,
- 5-chloro-2-(5-chlorothiophene-2-sulfonylamino-N-(4-(morpholine-4-sulfonyl)-phenyl)-benzamide sodium salt (6), described in WO00/02851,
- 2-(4-chloro-phenylsulfonylamino)-4,5-dimethoxy-N-(4-(thiomorpholine-4-sulfonyl)-phenyl)-benzamide (7), described in WO00/02851,
- 1-{6-[5-chloro-2-({4-trans-4-}trifluoromethyl)cyclohexyl]benzyl}oxy)phenyl] pyridin-2-yl}-5-(trifluoromethyl)-1H-pyrazole-4-carboxylic acid (8), described in WO 2009/032249,
- 1-[6-(2-(2-methyl-4-(4-trifluoromethoxyphenyl)benzyloxy)-phenyl)pyridine-2-yl]-5-trifluoromethyl-pyrazole-4-carboxylic acid (9), described in WO 2009/071504,
- 1[6-(3,4-dichlorophenyl)-2-pyridinyl-5-(trifluoromethyl)-1H-pyrazole-4-carboxylic acid (10), described in WO 2009/068652,
- 1-({2-[3-chloro-5-(trifluoromethyl)phenyl]-5-methyl-1,3-thiazol-4-yl}methyl)-1H-pyrazole-4-carboxylic acid (11), 4-({2-[3-(trifluoromethyl)phenyl]-1,3-thiazol-4-yl}methyl)benzoic acid (12) and 1-({2-[2-fluoro-3-(trifluoromethyl)phenyl]-5-methyl-1,3-thiazol-4-yl}methyl)-1H-pyrazole-4-carboxylic acid (13) described in WO 2009/123316.

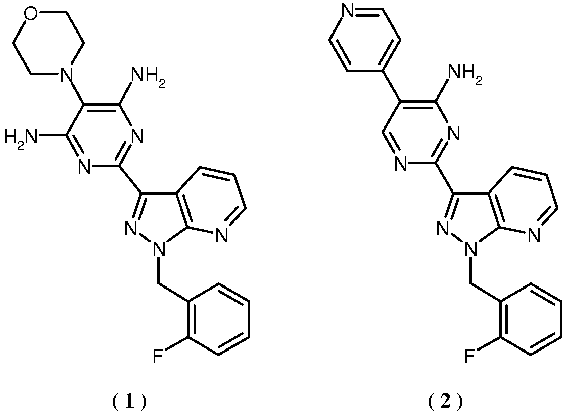

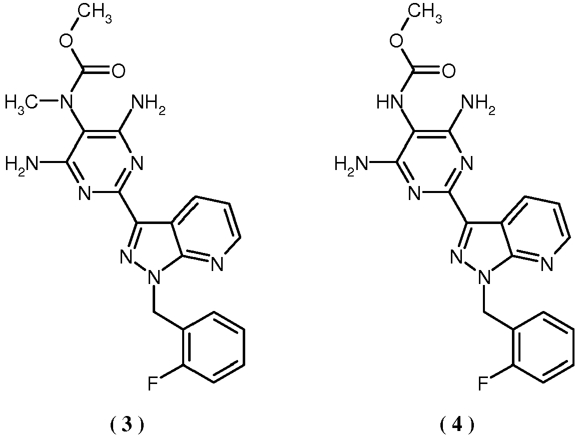

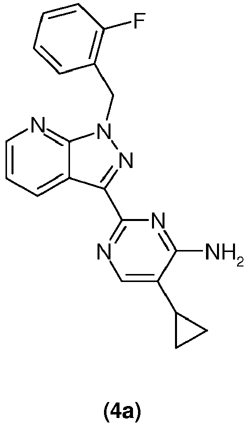

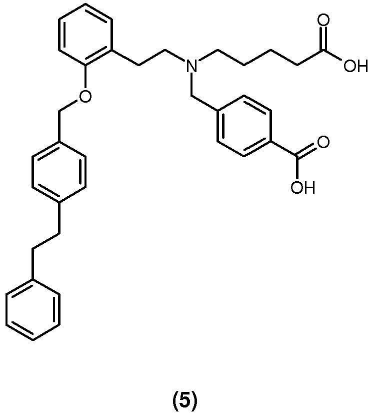

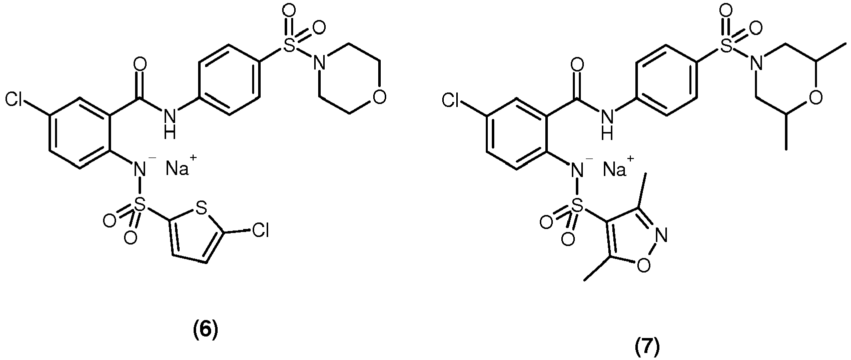

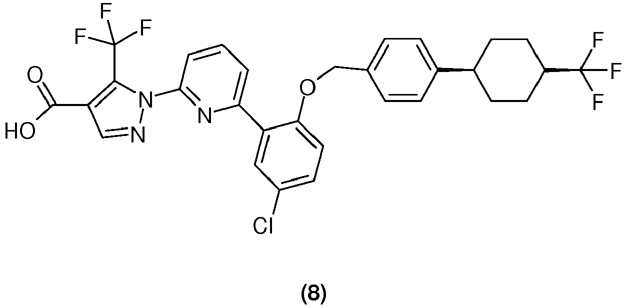

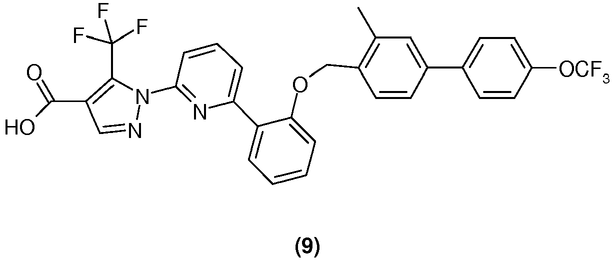

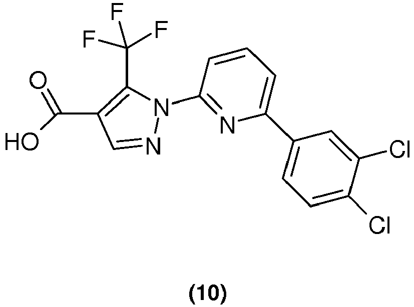

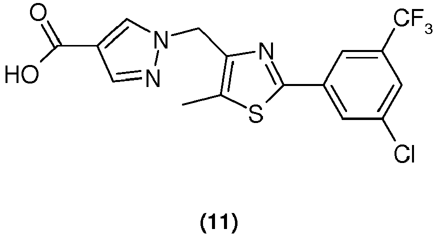

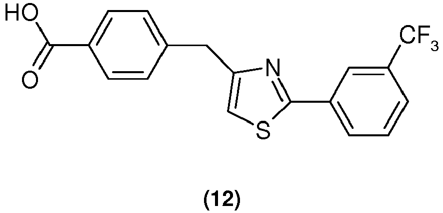

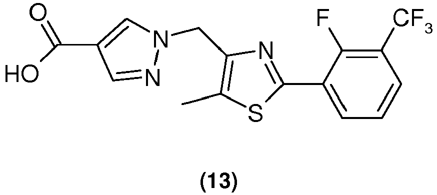

Compounds (1), (2), (3), (4) and (4a) are known soluble guanylate cyclase (sGC) stimulators which have been previously described for the treatment of stable angina pectoris or erectile dysfunction.

Compounds (5), (6), (7), (8), (9), (10), (11), (12) and (13) are known as sGC activators. PDE-5 inhibitors which are useful for the combined treatment Cystic Fibrosis are in particular *Tadalafil* ((6R,12aR) -2,3,6,7,12,12a - Hexahydro - 2 - methyl - 6 - (3,4-methylene -dioxyphenyl) pyrazino(1',2':1,6) pyrido(3,4-b)indole-1,4-dione), *Vardenafil* (2-(2-Ethoxy-5-(4-ethylpiperazin-1-yl-1-sulfonyl)phenyl)-5-methyl-7-propyl-3H-imidazo (5,1-f) (1,2,4)triazin-4-one), *Sildenafil* (3-[2-ethoxy-5-(4-methylpiperazin-1-yl)sulfonyl-phenyl]- 7-methy I- 9- propy I-2,4,7,8- tetrazabicyclo [4.3.0]nona -3,8,10-trien-5-one), *Udenafil* 5-[2-propyloxy-5-(1-methyl-2-pyrrolidinylethylamidosulfonyl)phenyl]-methyl-3-propyl-1,6-dihydro-7H-pyrazolo(4,3-d)pyrimidine-7-one, *Dasantafil* 7-(3-Bromo-4-methoxybenzyl)-1-ethyl-8-[[(1,2)-2-hydroxycyclopentyl]amino]-3-(2-hydroxyethyl)-3,7-dihydro-1-purine-2,6-dione, *Avanafil* 4-{[(3-chloro-4-methoxyphenyl)methyl]amino}-2-[(2S)-2-(hydroxymethyl)pyrrolidin-1-yl]-N-(pyrimidin-2-ylmethyl)pyrimidine-5-carboxamide, Mirodenafil, Lodenafil, UK 369.003, UK 371.800, *SLx 2101* of Surface Logix, *LAS 34179*Triazolo[1,2-]xanthine,6-methyl-4-propyl-2-[2-propoxy-5-(4-methylpiperazino)sulfonyl]phenyl-, or salts, hydrates or hydrates of salts of the before mentioned PDE5 inhibitors.

A pharmaceutical composition of the invention is formulated to be compatible with its intended route of administration. Examples of routes of administration include parenteral e.g., intravenous, intradermal, subcutaneous' oral (e.g.' inhalation)' transdermal (topical) transmucosal and rectal administration. Pharmaceutical compositions suitable for injectable use include sterile aqueous solutions (where water soluble) or dispersions and sterile powders for the extemporaneous preparation of sterile injectable solutions or dispersions. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents for example, parabens, chlorobutanol, phenol, ascorbic acid, thimerosal, and the like. In many cases, it will be preferable to include isotonic agents, for example, sugars, polyalcohols such as maitol sorbitol sodium chloride in the composition.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or con1 starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g.' a gas such as carbon dioxide, or a nebulizer.

Systemic administration can also be by transmucosal or transderrnal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Bio degradable, biocompatible polymers can be used, such as ethylene vinyl acetate, polyanhydrides, polyglycolic acid, collagen, polyorthoesters, and polylactic acid.

In another embodiment the invention provides sGC stimulators or sGC activators in combination with PDE5 inhbitiors and the use of sGC stimulators or sGC activators in combination with PDE5 inhbitiors for the preparation of pharmaceutical compositions for Cystic Fibrosis (CF), whereby these combinations comprise either i) pharmaceutical compositions comprising a compound having a sGC stimulatory or activatory action and PDE-5 inhibitory activity, or ii) pharmaceutical compositions comprising one sGC stimulator and sGC activator and at least one PDE-5 inhibitor as a fixed combination in one application unit, or iii) a kit of parts containing at least two sets of pharmaceutical compositions, each set consisting of at least one pharmaceutical preparation comprising a PDE-5 inhibitor in units of at least one dose and at least one pharmaceutical preparation comprising a sGC activator or sGC stimulator in units of at least one dose, whereby each application unit of said pharmaceutical compositions is administered in combination, sequentially, as single dose or in multiple doses.

### In particular, the present invention provides:

A pharmaceutical composition for use in the treatment of a disease comprised in a group of diseases consisting of Cystic Fibrosis (CF), containing at least one compound selected from
2-[1-(2-fluorobenzyl)-1 H-pyrazolo[3,4-b]pyridine-3-yl]-5-(4-morpholinyl)-4,6-pyrimidinediamine (1),
2-[1-(2-fluorobenzyl)-1H-pyrazolo[3,4-b]pyridine-3-yl]-5-(4-pyridinyl)-4-pyrimidinamine (2),
methyl-4,6-diamino-2-[1-(2-fluorobenzyl)-1H-pyrazolo[3,4-b]pyridine-3-yl]-5-pyrimidinyl-(methyl)carbamate (3), and
methyl-4,6-diamino-2-[1-(2-fluorobenzyl)-1H-pyrazolo[3,4-b]pyridine-3-yl]-5-pyrimidinyl-carbamate (4),
3-(4-amino-5-cyclopropylpyrimidine-2-yl)-1-(2-fluorobenzyl)1 H-pyrazolo[3,4-b]pyridine (4a),
and
5-chloro-2-(5-chlorothiophene-2-sulfonylamino-N-(4-(morpholine-4-sulfonyl)-phenyl)-benzamide sodium salt (6),
2-(4-chloro-phenylsulfonylamino)-4,5-dimethoxy-N-(4-(thiomorpholine-4-sulfonyl)-phenyl)-benzamide (7),
and
4-({(4-carboxybutyl)[2-(2-{[4-(2-phenylethyl)benzyl]oxy}phenyl)ethyl]amino}methyl) benzoic acid (5)
1-{6-[5-chloro-2-({4-trans-4-}trifluoromethyl)cyclohexyl]benzyl}oxy)phenyl]pyridin-2-yl}-5-(trifluoromethyl)-1 H-pyrazole-4-carboxylic acid (8)
1-[6-(2-(2-methyl-4-(4-trifluoromethoxyphenyl)benzyloxy)-phenyl)pyridin-2-yl]-5-trifluoromethyl-pyrazole-4-carboxylic acid (9)
1[6-(3,4-dichlorophenyl)-2-pyridinyl-5-(trifluoromethyl)-1H-pyrazole-4-carboxylic acid (10) 1-({2-[3-chloro-5-(trifluoromethyl)phenyl]-5-methyl-1,3-thiazol-4-yl}methyl)-1H-pyrazole-4-carboxylic acid (11)
4-({2-[3-(trifluoromethyl)phenyl]-1,3-thiazol-4-yl}methyl)benzoic acid (12)
1-({2-[2-fluoro-3-(trifluoromethyl)phenyl]-5-methyl-1,3-thiazol-4-yl}methyl)-1H-pyrazole-4-carboxylic acid (13)
and at least one compound selected from
*Tadalafil* ((6R,12aR) -2,3,6,7,12,12a - Hexahydro - 2 - methyl - 6 - (3,4-methylene - dioxyphenyl) pyrazino(1',2':1,6) pyrido(3,4-b)indole-1,4-dione), *Vardenafil* (2-(2-Ethoxy-5-(4-ethylpiperazin-1-yl-1-sulfonyl)phenyl)-5-methyl-7-propyl-3H-imidazo (5,1-f) (1,2,4)triazin-4-one), *Sildenafil* (3-[2-ethoxy-5-(4-methylpiperazin-1-yl)sulfonyl-phenyl]-7-methy-I-9-propyl-2,4,7,8-tetrazabicyclo [4.3.0]nona-3,8,10-trien-5-one), *Udenafil* 5-[2-propyloxy-5-(1-methyl-2-pyrrolidinyl-ethyl-amidosulfonyl)phenyl]-methyl-3-propyl-1,6-dihydro-7H-pyrazolo(4,3-d)pyrimidine-7-one, *Dasantafil* 7-(3-Bromo-4-methoxybenzyl)-1-ethyl-8-[[(1,2)-2-hydroxycyclopentyl]amino]-3-(2-hydroxyethyl)-3,7-dihydro-1-purine-2,6-dione, *Avanafil* 4-{[(3-chloro-4-methoxy phenyl)methyl]amino}-2-[(2S)-2-(hydroxymethyl)pyrrolidin-1-yl]-N-(pyrimidin-2-yl methyl)pyrimidine-5-carboxamide, Mirodenafil, Lodenafil, UK 369.003, UK 371.800, *SLx 2101* of Surface Logix, and/or *LAS* 34179Triazolo[1,2-]xanthine,6-methyl-4-propyl-2-[2-propoxy-5-(4-methylpiperazino)-sulfonyl]phenyl or salts, hydrates or hydrates of salts of the before mentioned PDE5 inhibitors.

Further disclosed is a pharmaceutical composition for the use in the treatment of a disease comprised in a group of diseases in which altered CFTR-function is causally involved, consisting but not limited to pancreatic disorders, gastrointestinal disorders, liver disorders, Cystic Fibrosis-related diabetes (CFRD), dry eye, dry mouth, Sjoegren's syndrome, containing at least one compound selected from
2-[1-(2-fluorobenzyl)-1 H-pyrazolo[3,4-b]pyridine-3-yl]-5-(4-morpholinyl)-4,6-pyrimidinediamine (1),
2-[1-(2-fluorobenzyl)-1H-pyrazolo[3,4-b]pyridine-3-yl]-5-(4-pyridinyl)-4-pyrimidinamine (2),
methyl-4,6-diamino-2-[1-(2-fluorobenzyl)-1 H-pyrazolo[3,4-b]pyridin-3-yl]-5-pyrimidinyl-(methyl)carbamate (3), and
methyl-4,6-diamino-2-[1-(2-fluorobenzyl)-1H-pyrazolo[3,4-b]pyridin-3-yl]-5-pyrimidinyl-carbamate (4),
3-(4-amino-5-cyclopropylpyrimidine-2-yl)-1-(2-fluorobenzyl)1 H-pyrazolo[3,4-b]pyridine (4a),
and
5-chloro-2-(5-chlorothiophene-2-sulfonylamino-N-(4-(morpholine-4-sulfonyl)-phenyl)-benzamide sodium salt (6),
2-(4-chloro-phenylsulfonylamino)-4,5-dimethoxy-N-(4-(thiomorpholine-4-sulfonyl)-phenyl)-benzamide (7),
and
4-({(4-carboxybutyl)[2-(2-{[4-(2-phenylethyl) benzyl] oxy}phenyl)ethyl]amino}methyl) benzoic acid (5)
1-{6-[5-chloro-2-({4-trans-4-}trifluoromethyl)cyclohexyl]benzyl}oxy)phenyl]pyridin-2-yl}-5-(trifluoromethyl)-1 H-pyrazole-4-carboxylic acid (8)
1-[6-(2-(2-methyl-4-(4-trifluoromethoxyphenyl)benzyloxy)-phenyl)pyridin-2-yl]-5-trifluoromethyl-pyrazole-4-carboxylic acid (9)
1[6-(3,4-dichlorophenyl)-2-pyridinyl-5-(trifluoromethyl)-1H-pyrazole-4-carboxylic acid (10)
1-({2-[3-chloro-5-(trifluoromethyl)phenyl]-5-methyl-1,3-thiazol-4-yl}methyl)-1H-pyrazole-4-carboxylic acid (11)
4-({2-[3-(trifluoromethyl)phenyl]-1,3-thiazol-4-yl}methyl)benzoic acid (12)
1-({2-[2-fluoro-3-(trifluoromethyl)phenyl]-5-methyl-1,3-thiazol-4-yl}methyl)-1H-pyrazole-4-carboxylic acid (13)
and at least one compound selected from
*Tadalafil* ((6R,12aR) -2,3,6,7,12,12a - Hexahydro - 2 - methyl - 6 - (3,4-methylene - dioxyphenyl) pyrazino(1',2':1,6) pyrido(3,4-b)indole-1,4-dione), *Vardenafil* (2-(2-Ethoxy-5-(4-ethylpiperazin-1-yl-1-sulfonyl)phenyl)-5-methyl-7-propyl-3H-imidazo (5,1-f) (1,2,4)triazin-4-one), *Sildenafil* (3-[2-ethoxy-5-(4-methylpiperazin-1-yl)sulfonyl-phenyl]-7-methy-I-9-propyl-2,4,7,8-tetrazabicyclo [4.3.0]nona-3,8,10-trien-5-one), *Udenafil* 5-[2-propyloxy-5-(1-methyl-2-pyrrolidinyl-ethyl-amidosulfonyl)phenyl]-methyl-3-propyl-1,6-dihydro-7H-pyrazolo(4,3-d)pyrimidine-7-one, *Dasantafil* 7-(3-Bromo-4-methoxybenzyl)-1-ethyl-8-[[(1,2)-2-hydroxycyclopentyl]amino]-3-(2-hydroxyethyl)-3,7-dihydro-1-purine-2,6-dione, *Avanafil* 4-{[(3-chloro-4-methoxy phenyl)methyl]amino}-2-[(2S)-2-(hydroxymethyl)pyrrolidin-1-yl]-N-(pyrimidin-2-yl methyl)pyrimidine-5-carboxamide, Mirodenafil, Lodenafil, UK 369.003, UK 371.800, *SLx 2101* of Surface Logix, and/or *LAS 34179*Triazolo[1,2-]xanthine,6-methyl-4-propyl-2-[2-propoxy-5-(4-methylpiperazino)-sulfonyl]phenyl or salts, hydrates or hydrates of salts of the before mentioned PDE5 inhibitors.Use of a sGC stimulator and activator for the preparation of a pharmaceutical composition for the treatment of Cystic Fibrosis (CF).

Further disclosed is a use of a sGC stimulator and activator for the preparation of a pharmaceutical composition for the treatment of pancreatic disorders, gastronintestinal disorders, liver disorders, Cystic Fibrosis-related diabetes (CFRD), dry eye, dry mouth, Sjoegren's syndrome.

Use of a combination of at least one sGC stimulator or activator and at least one PDE5 inhibitor for the preparation of a pharmaceutical composition for the treatment of Cystic Fibrosis (CF).

Further disclosed is a use of a combination of at least one sGC stimulator or activator and at least one PDE5 inhibitor for the preparation of a pharmaceutical composition for the treatment of pancreatic disorders, liver disorders, Cystic Fibrosis-related diabetes (CFRD), dry eye, dry mouth, Sjoegren's syndrome.

Use of sGC stimulator or activator selected from the group of sGC stimulators and activators of 2-[1-(2-fluorobenzyl)-1H-pyrazolo[3,4-b]pyridin-3-yl]-5-(4-morpholinyl)-4,6-pyrimidinediamine(1), 2-[1-(2-fluorobenzyl)-1H-pyrazolo[3,4-b]pyridin-3-yl]-5-(4-pyridinyl)-4-pyrimidinamine (2), methyl-4,6-diamino-2-[1-(2-fluorobenzyl)-1H-pyrazolo[3,4-b]pyridin-3-yl]-5-pyrimidinyl(methyl)carbamate (3), methyl-4,6-diamino-2-[1-(2-fluorobenzyl)-1H-pyrazolo[3,4-b]pyridin-3-yl]-5-pyrimidinylcarbamate (4), 5-chloro-2-(5-chlorothiophene-2-sulfonylamino-N-(4-(morpholine-4-sulfonyl)-phenyl)-benzamide sodium salt (6), 2-(4-chloro-phenylsulfonylamino)-4,5-dimethoxy-N-(4-(thiomorpholine-4-sulfonyl)-phenyl)-benzamide (7), 4-({(4-carboxybutyl)[2-(2-{[4-(2-phenylethyl) benzyl] oxy}phenyl)ethyl]amino}methyl) benzoic acid (5), 1-{6-[5-chloro-2-({4-trans-4-}trifluoromethyl)cyclohexyl]benzyl}oxy)phenyl] pyridin-2-yl}-5-(trifluoromethyl)-1H-pyrazole-4-carboxylic acid (8), 1-[6-(2-(2-methyl-4-(4-trifluoromethoxyphenyl)benzyloxy)-phenyl)pyridin-2-yl]-5-trifluoromethyl-pyrazole-4-carboxylic acid (9), 1[6-(3,4-dichlorophenyl)-2-pyridinyl-5-(trifluoromethyl)-1H-pyrazole-4-carboxylic acid (10), 1-({2-[3-chloro-5-(trifluoromethyl)phenyl]-5-methyl-1,3-thiazol-4-yl}methyl)-1 H-pyrazole-4-carboxylic acid (11), 4-({2-[3-(trifluoromethyl)phenyl]-1,3-thiazol-4-yl}methyl)benzoic acid (12), 1-({2-[2-fluoro-3-(trifluoromethyl)phenyl]-5-methyl-1,3-thiazol-4-yl}methyl)-1H-pyrazole-4-carboxylic acid (13) for the preparation of a pharmaceutical composition for the treatment of Cystic Fibrosis (CF).

Use of a combination of at least one sGC stimulator and activator selected from the group of sGC stimulators and activators of 2-[1-(2-fluorobenzyl)-1 H-pyrazolo[3,4-b]pyridine-3-yl]-5-(4-morpholinyl)-4,6-pyrimidinediamine (1), 2-[1-(2-fluorobenzyl)-1 H-pyrazolo[3,4-b]pyridine-3-yl]-5-(4-pyridinyl)-4-pyrimidinamine (2), methyl-4,6-diamino-2-[1-(2-fluorobenzyl)-1 H-pyrazolo[3,4-b]pyridine-3-yl]-5-pyrimidinyl(methyl)carbamate (3), methyl-4,6-diamino-2-[1-(2-fluorobenzyl)-1 H-pyrazolo[3,4-b]pyridin-3-yl]-5-pyrimidinylcarbamate (4), 3-(4-amino-5-cyclopropylpyrimidine-2-yl)-1-(2-fluorobenzyl)1H-pyrazolo[3,4-b]pyridine (4a), 5-chloro-2-(5-chlorothiophene-2-sulfonylamino-N-(4-(morpholine-4-sulfonyl)-phenyl)-benzamide sodium salt (6), 2-(4-chloro-phenylsulfonylamino)-4,5-dimethoxy-N-(4-(thiomorpholine-4-sulfonyl)-phenyl)-benzamide (7), and 4-({(4-carboxybutyl)[2-(2-{[4-(2-phenylethyl) benzyl] oxylphenyl)ethyl]amino}methyl) benzoic acid (5), 1-{6-[5-chloro-2-({4-trans-4-}trifluoromethyl)cyclohexyl]benzyl}oxy)phenyl]pyridin-2-yl}-5-(trifluoromethyl)-1H-pyrazole-4-carboxylic acid (8), 1-[6-(2-(2-methyl-4-(4-trifluoromethoxyphenyl)benzyloxy)-phenyl)pyridin-2-yl]-5-trifluoromethyl-pyrazole-4-carboxylic acid (9), 1[6-(3,4-dichlorophenyl)-2-pyridinyl-5-(trifluoromethyl)-1 H-pyrazole-4-carboxylic acid (10), 1-({2-[3-chloro-5-(trifluoromethyl)phenyl]-5-methyl-1,3-thiazol-4-yl}methyl)-1H-pyrazole-4-carboxylic acid (11), 4-({2-[3-(trifluoromethyl)phenyl]-1,3-thiazol-4-yl}methyl)benzoic acid (12), 1-({2-[2-fluoro-3-(trifluoromethyl)phenyl]-5-methyl-1,3-thiazol-4-yl}methyl)-1H-pyrazole-4-carboxylic acid (13)
and at least one PDE-5 inhibitor selected from the group of PDE-5 inhibitors consisting of Vardenafil (2-(2-Ethoxy-5-(4-ethylpiperazin-1-yl-1-sulfonyl)phenyl)-5-methyl-7-propyl-3H-imidazo (5,1-f) (1,2,4) triazin-4-one), Sildenafil (3-[2-ethoxy-5-(4-methylpiperazin-1-yl)sulfonyl-phenyl] - 7- methy I- 9- propy I-2,4,7,8- tetrazabicyclo [4.3.0]nona -3,8,10-trien-5-one), and Tadalafil ((6R,12aR) -2,3,6,7,12,12a- Hexahydro -2-methyl-6- (3,4-methylene-dioxyphenyl) for the preparation of a pharmaceutical composition for the treatment of Cystic Fibrosis (CF).

Further disclosed is a method for the preparation of a pharmaceutical composition for the treatment of the diseases as mentioned above wherein stimulator and activator of the soluble guanylate-cyclase is selected from the group of compounds consisting of 2-[1-(2-fluorobenzyl)-1H-pyrazolo[3,4-b]pyridine-3-yl]-5-(4-morpholinyl)-4,6-pyrimidinediamine (1), 2-[1-(2-fluorobenzyl)-1H-pyrazolo[3,4-b]pyridine-3-yl]-5-(4-pyridinyl)-4-pyrimidinamine (2), methyl-4,6-diamino-2-[1-(2-fluorobenzyl)-1H-pyrazolo[3,4-b]pyridine-3-yl]-5-pyrimidinyl-(methyl)carbamate (3), methyl-4,6-diamino-2-[1-(2-fluorobenzyl)-1H-pyrazolo[3,4-b]pyridine-3-yl]-5-pyrimidinylcarbamate (4), 3-(4-amino-5-cyclopropylpyrimidine-2-yl)-1-(2-fluorobenzyl)1 H-pyrazolo[3,4-b]pyridine (4a), 5-chloro-2-(5-chlorothiophene-2-sulfonylamino-N-(4-(morpholine-4-sulfonyl)-phenyl)-benzamide sodium salt (6), 2-(4-chloro-phenylsulfonylamino)-4,5-dimethoxy-N-(4-(thiomorpholine-4-sulfonyl)-phenyl)-benzamide (7),
4-({(4-carboxybutyl)[2-(2-{[4-(2-phenylethyl) benzyl] oxy}phenyl)ethyl]amino}methyl) benzoic acid (5), 1-{6-[5-chloro-2-({4-trans-4-}-trifluoromethyl)cyclohexyl]benzyl}oxy)phenyl]pyridin-2-yl}-5-(trifluoromethyl)-1H-pyrazole-4-carboxylic acid (8), 1-[6-(2-(2-methyl-4-(4-trifluoromethoxyphenyl)benzyloxy)-phenyl)pyridin-2-yl]-5-trifluoromethyl-pyrazole-4-carboxylic acid (9), 1[6-(3,4-dichlorophenyl)-2-pyridinyl-5-(trifluoromethyl)-1 H-pyrazole-4-carboxylic acid (10), 1-({2-[3-chloro-5-(trifluoromethyl)phenyl]-5-methyl-1,3-thiazol-4-yl}methyl)-1H-pyrazole-4-carboxylic acid (11), 4-({2-[3-(trifluoromethyl)phenyl]-1,3-thiazol-4-yl}methyl)benzoic acid (12), 1-({2-[2-fluoro-3-(trifluoromethyl)phenyl]-5-methyl-1,3-thiazol-4-yl}methyl)-1H-pyrazole-4-carboxylic acid (13).

Use of a pharmaceutlical composition as mentioned above for the stimulation and activation of the soluble guanylate cyclase in a mammal having Cystic Fibrosis (CF).

Use of a pharmaceutical composition as mentioned above for the stimulation and activation of the soluble guanylate cyclase and for the regulation of PDE activity in a mammal having Cystic Fibrosis (CF).

A pharmaceutical composition containing at least methyl-4,6-diamino-2-[1-(2-fluorobenzyl)-1H-pyrazolo[3,4-b]pyridine-3-yl]-5-pyrimidinylcarbamate (4) and/or 4-({(4-carboxybutyl)[2-(2-{[4-(2-phenylethyl) benzyl]oxy}phenyl)ethyl]amino}methyl)benzoic acid (5) and at least Sildenafil, Tadalafil, Udenafil, Dasantafil, Avanafil, Mirodenafil, Lodenafil, UK 369.003, UK 371.800, SLx2101 and LAS34179 and preferably Vardenafil or a salt, a hydrat or a hydrat of a salt of the before mentioned PDE5 inhibitors, for the treatment of Cystic Fibrosis (CF).

In order to clarify the effect of sGC stimulators and sGC activators alone and in combination with vardenafil experiments are performed.

In particular sGC stimulators and sGC activators, i.e. methyl-4,6-diamino-2-[1-(2-fluorobenzyl)-1H-pyrazolo[3,4-b]pyridine-3-yl]-5-pyrimidinylcarbamate (4) alone and in combination with PDE5 inhibitors, i.e. Vardenafil were tested in vitro and in vivo:
- in BHK-cells expressing a mutant CFTR channel, i.e. a tagged deltaF508 CFTR channel, on cell surface expression of the delta F508 CFTR.
- in HEK-293 cells expressing a mutant CFTR channel, i.e. a deltaF508CFTR, on CFTR function via measurement of iodine influx through fluoresence.
- in HEK-293 cells expressing a mutant CFTR channel, i.e. a deltaF508CFTR, on enhanced biosynthesis (band B) and processing (band C).
- in CFBE410⁻ cells expressing a mutant CFTR channel, i.e. a delta F508CFTR, on transepithelial conductance in ussing chamber experiments.
- in rectal biopsies of CF-patients and compared with rectal biopsies of non-CF patients on transepithelial conductance in using chamber experiments.
- in transgenic mice expressing the delta F508CFTR channel, on nasal potential difference and salivation.
- in transgenic mice not expressing the CFTR, on nasal potential difference and salivation.

Combinations of sGC stimulators and activators, i.e. methyl-4,6-diamino-2-[1-(2-fluorobenzyl)-1 H-pyrazolo[3,4-b]pyridine-3-yl]-5-pyrimidinylcarbamate (4) with PDE5 inhibitors, i.e. vardenafil are safe, with a hemodynamic profile similar to vardenafil.

The preferred embodiment of the invention is a combination of at least one sGC stimulator or activator selected from the group comprising of 2-[1-(2-fluorobenzyl)-1H-pyrazolo[3,4-b]pyridine-3-yl]-5-(4-morpholinyl)-4,6-pyrimidinediamine (1), 2-[1-(2-fluorobenzyl)-1 H-pyrazolo[3,4-b]pyridine-3-yl]-5-(4-pyridinyl)-4-pyrimidinamine (2), methyl-4,6-diamino-2-[1-(2-fluorobenzyl)-1H-pyrazolo[3,4-b]pyridine-3-yl]-5-pyrimidinyl-(methyl)carbamate (3), methyl-4,6-diamino-2-[1-(2-fluorobenzyl)-1H-pyrazolo[3,4-b]pyridin-3-yl]-5-pyrimidinylcarbamate (4), 3-(4-amino-5-cyclopropylpyrimidine-2-yl)-1-(2-fluorobenzyl)1 H-pyrazolo[3,4-b]pyridine (4a), 5-chloro-2-(5-chlorothiophene-2-sulfonylamino-N-(4-(morpholine-4-sulfonyl)-phenyl)-benzamide sodium salt (6), 2-(4-chloro-phenylsulfonylamino)-4,5-dimethoxy-N-(4-(thiomorpholine-4-sulfonyl)-phenyl)-benzamide (7), and/or 4-({(4-carboxybutyl)[2-(2-{[4-(2-phenylethyl) benzyl] oxy}phenyl)ethyl]amino}methyl) benzoic acid (5), 1-{6-[5-chloro-2-({4-trans-4-}trifluoromethyl)cyclohexyl]benzyl}oxy)phenyl]pyridin-2-yl}-5-(trifluoromethyl)-1H-pyrazole-4-carboxylic acid (8), 1-[6-(2-(2-methyl-4-(4-trifluoromethoxyphenyl)benzyloxy)-phenyl)pyridin-2-yl]-5-trifluoromethyl-pyrazole-4-carboxylic acid (9), 1[6-(3,4-dichlorophenyl)-2-pyridinyl-5-(trifluoromethyl)-1 H-pyrazole-4-carboxylic acid (10), 1-({2-[3-chloro-5-(trifluoromethyl)phenyl]-5-methyl-1,3-thiazol-4-yl}methyl)-1H-pyrazole-4-carboxylic acid (11), 4-({2-[3-(trifluoromethyl)phenyl]-1,3-thiazol-4-yl}methyl)benzoic acid (12), 1-({2-[2-fluoro-3-(trifluoromethyl)phenyl]-5-methyl-1,3-thiazol-4-yl}methyl)-1H-pyrazole-4-carboxylic acid (13) with a PDE5 inhibitor selected form the group comprising Vardenafil, Sildenafil, Tadalafil, Udenafil, Dasantafil, Avanafil, Mirodenafil, Lodenafil, UK 369.003, UK 371.800, SLx2101 and LAS34179 in which the combination comprises 0.1 to 1 mg of the sGC stimuulator or activator and 2.5 to 20 mg of the PDE5 inhibitor.

Another preferred embodiment of the invention is a combination according to claim 1 in which the sGC stimulator is methyl-4,6-diamino-2-[1-(2-fluorobenzyl)-1 H-pyrazolo[3,4-b]pyridine-3-yl]-5-pyrimidinyl(methyl)carbamate (3) or methyl-4,6-diamino-2-[1-(2-fluorobenzyl)-1 H-pyrazolo[3,4-b]pyridine-3-yl]-5-pyrimidinylcarbamate (4).

Another preferred embodiment of the invention is a combination as disclosed above in which the PDE5 inhibitor is Vardenafil or Sildenafil.

Another preferred embodiment of the invention is a combination as disclosed above in which the sGC stimulator is methyl-4,6-diamino-2-[1-(2-fluorobenzyl)-1 H-pyrazolo[3,4-b]pyridine-3-yl]-5-pyrimidinyl(methyl)carbamate (3) or methyl-4,6-diamino-2-[1-(2-fluorobenzyl)-1H-pyrazolo[3,4-b]pyridine-3-yl]-5-pyrimidinylcarbamate (4) and the PDE5 inhibitor is Vardenafil.

Another preferred embodiment of the invention is a combination according to the embodiments disclosed above for the use as a medicament.

Another preferred embodiment of the invention is the use of a combination as disclosed above for the manufacture fo a medicament for the treatment of Cystic Fibrosis (CF).

Another preferred embodiment of the invention is a pharmaceutical formulation comprising at least one combination as disclosed above.

Another preferred embodiment of the invention is a pharmaceutical formulation comprising at least one combination as disclosed above for the use in Cystic Fibrosis (CF).

### Table 1:

Salivation rates are expressed as the mean percentage increase after treatment with vardenafil (0.07, 0.14, 0.42 mg/kg i.p.) and BAY 41-2272 (compound according to formula (4a)) (0.01, 0.03, 0.1, 0.3 mg/kg i.p.) compared to placebo treatment, */**/*** means statistical significant with p values <0.05/<0.01/0.001 analyzed by one way ANOVA test followed by post-hoc Bonferoni analysis.
Table 1

**Table 1: Salivation rates in deltaF508 CFTR mice in percent of placebo**

| | Salivation rate [%] | Statistical Significance | number of mice [n] |
|---|---|---|---|
| Placebo | 100 | | 49 |
| Vardenafil - 0.07 mg/kg | 111 | n.s. | 25 |
| Vardenafil - 0.14 mg/kg | 215 | *** | 39 |
| Vardenafil - 0.42 mg/kg | 202 | *** | 30 |
| BAY 41-2272 - 0.01 mg/kg | 142 | n.s. | 40 |
| BAY 41-2272 - 0.03 mg/kg | 164 | ** | 59 |
| BAY 41-2272 - 0.10 mg/kg | 179 | *** | 59 |
| BAY 41-2272 - 0.30 mg/kg | 200 | *** | 40 |

### Example1:

Effects of vardenafil and the sGC stimulator in transgenic mice expressing the delta F508 CFTR channel, on salivation.

### Salivary Secretion Assay in delta(Δ)508-CFTR mice

Male and female homozygous, heterozygous Δ508-CFTR (backcrossed on the FVB genetic background for more than 12 generations, originally obtained from Erasmus University, Rotterdam; van Doorninck et al., 1995), 10-14 weeks old and weighing 18-36g of both sexes were used in this assay.

Solutions of Vardenafil in concentrations of 0.07, 0.14 and 0.42 mg/kg BW were prepared in sterile saline, whereas the sGC stimulator BAY 41-2272 was dissolved to 0.01, 0.03, 0.1 and 0.3 mg/kg BW in a solvent containing 50% ddH₂O, 40% PEG 400 (polyethylene glycol 400) and 10% ethanol. The substances or the appropriate vehicles were administered to mice via intraperitoneal injection (5 ml/kg BW) 60 min prior to the salivary secretion assay. After 60 min, mice were anaesthetized with a combination of ketamine and diazepam. The solution was prepared to contain 1 ml of 5 mg/ml diazepam and 1 ml of 100 mg/ml ketamine in 8 ml sterile saline. Anaesthesia was induced by intraperitoneal injection of the solution (10 ml/kg BW). After anaesthesia, mice were pretreated with a subcutaneous injection of 1 mM atropine (50 µl) into the left cheek in order to avoid a cross-stimulation of cholinergic receptors. Small strips of Whatman filter paper were placed inside the previously injected cheek for 4 min to absorb any saliva secreted after the injection of atropine. This first piece of filter paper was removed and replaced with a second pre-weighed filter paper. Thereafter, 50 µl of a solution containing 100 µM isoprenaline and 1 mM atropine was injected into the left cheek at the same site to induce the salivary secretion by adrenergic mechanisms. The time of the isoprenaline injection was taken as time zero, and filter paper stripes were replaced every 10 minutes for a total collection period of 30 minutes. Each piece of filter paper was immediately placed and sealed in a pre-weighed vial. After all samples had been collected, each vial was remeasured and the weights of all samples were recorded. The difference in total weight of vial plus paper measured before and after collecting saliva was taken as the net weight of saliva secreted during the collection period. The total amounts of salivary secretion were calculated as the weight of saliva divided by the number of minutes required for each collection and then normalized to the mass of the mouse in grams. Results are expressed in table 1 as the mean percentage increase of n mice compared to placebo treatment. Statistics was analyzed by one way ANOVA test followed by post-hoc Bonferoni analysis; */**/*** means statistical significant with p values <0.05/<0.01/0.001 and n.s. means non significant.

### References:

Carlile GW, Robert R, Zhang D (2007): Correctors of protein trafficking defects identified by a novel high-throughput screening assay. Chem Bio Chem 8: 1012-1020
Clarke LL (2008): Phosphodiesterase 5 Inhibitors and Cystic Fibrosis. Am J RRespir Crit Care Med 177: 469-472
Cobb BR, Fan L, Kovacs TE (2003): Adenosine receptors and phosphodiesterase inhibitors stimulate Cl- secretion in Calu-3 cells. _Am J Respir Cell Mol Biol 29: 410-418
Dormer RL, Harris CM, Clark Z et al. (2004) : Sildenafil (Viagra) corrects ΔF508-CFTR location in nasal epithelia cells from patients with cystic fibrosis. Thorax 60: 55-59
Evgenov OV, Pacher P, Schmidt PM et al. (2006) NO-independent stimulators and activators of soluble guanylate cyclase: discovery and therapeutic potential. Nat Rev Drug Discov 5(9): 755-68
Lubamba B, Lecourt H, Lebacq J (2008): Preclinical evidence that sildenafil and vardenafil activate chloride transport in cystic fibrosis. Am J Respir Crit Care Med 177: 506-515
McPherson MA, Pereira MMC, Mills CL et al. (1999): A cyclic nucleotide PDE5 inhibitor corrects defective mucin secretion in submanidbular cells containing antibody directed against the cystic fibrosis transmembrane conductance regulator protein. FEBS Letters 464: 48-52
Robert R, Carlile GW, Pavel C et al. (2008): Structural analog of sildenafil identified as a novel corrector of the F508del-CFTR trafficking defect. Mol Pharmacol 73: 478-489.
Sandner P, Hütter J, Tinel H et al. (2007): PDE5 inhibitors beyond erectile dysfunction. Int J Impot Res 19(6): 533-543
Schmidt HHHW., Hoffmann F, Stasch JP, Editors (2009): cGMP: Generators, Effectors and therapeutic implications. Handbook of Experimental Pharmacology Vol. 191
Stasch JP, Becker EM, Alonso-Alija C et al. (2001): NO-independent regulatory site on soluble guanylate cyclase. Nature 8: 212-215.

## Claims

1. Use of a sGC stimulator or activator selected from the group comprising 2-[1-(2-fluorobenzyl)-1 H-pyrazolo[3,4-b]pyridine-3-yl]-5-(4-morpholinyl)-4,6-pyrimidinediamine (1), 2-[1-(2-fluorobenzyl)-1 H-pyrazolo[3,4-b]pyridine-3-yl]-5-(4-pyridinyl)-4-pyrimidinamine (2), methyl-4,6-diamino-2-[1-(2-fluorobenzyl)-1H-pyrazolo[3,4-b]pyridine-3-yl]-5-pyrimidinyl(methyl)carbamate (3), methyl-4,6-diamino-2-[1-(2-fluorobenzyl)-1 H-pyrazolo[3,4-b]pyridine-3-yl]-5-pyrimidinylcarbamate (4), 3-(4-amino-5-cyclopropylpyrimidine-2-yl)-1-(2-fluorobenzyl)1H-pyrazolo[3,4-b]pyridine (4a), 5-chloro-2-(5-chlorothiophene-2-sulfonylamino-N-(4-(morpholine-4-sulfonyl)-phenyl)-benzamide sodium salt (6), 2-(4-chloro-phenylsulfonylamino)-4,5-dimethoxy-N-(4-(thiomorpholine-4-sulfonyl)-phenyl)-benzamide (7), and/or 4-({(4-carboxybutyl)[2-(2-{[4-(2-phenylethyl) benzyl] oxy}phenyl)ethyl]amino}methyl) benzoic acid (5), 1-{6-[5-chloro-2-({4-trans-4-}trifluoromethyl)cyclohexyl]benzyl}oxy)phenyl] pyridin-2-yl}-5-(trifluoromethyl)-1H-pyrazole-4-carboxylic acid (8), 1-[6-(2-(2-methyl-4-(4-trifluoromethoxyphenyl)benzyloxy)-phenyl)pyridin-2-yl]-5-trifluoromethyl-pyrazole-4-carboxylic acid (9), 1[6-(3,4-dichlorophenyl)-2-pyridinyl-5-(trifluoromethyl)-1H-pyrazole-4-carboxylic acid (10), 1-({2-[3-chloro-5-(trifluoromethyl)phenyl]-5-methyl-1,3-thiazol-4-yl}methyl)-1H-pyrazole-4-carboxylic acid (11), 4-({2-[3-(trifluoromethyl)phenyl]-1,3-thiazol-4-yl}methyl)benzoic acid (12), 1-({2-[2-fluoro-3-(trifluoromethyl)phenyl]-5-methyl-1,3-thiazol-4-yl}methyl)-1H-pyrazole-4-carboxylic acid (13) for the manufacture of a medicament for the treatment of Cystic Fibrosis (CF).

2. Use according to claim 1 of a sGC stimulator selected from the group comprising methyl-4,6-diamino-2-[1-(2-fluorobenzyl)-1 H-pyrazolo[3,4-b]pyridine-3-yl]-5-pyrimidinyl(methyl)carbamate (3), methyl-4,6-diamino-2-[1-(2-fluorobenzyl)-1H-pyrazolo[3,4-b]pyridine-3-yl]-5-pyrimidinylcarbamate (4), 3-(4-amino-5-cyclopropylpyrimidine-2-yl)-1-(2-fluorobenzyl)1 H-pyrazolo[3,4-b]pyridine (4a) for the manufacture of a medicament for the treatment of Cystic Fibrosis (CF).

3. Use according to claims 1 and 2 of methyl-4,6-diamino-2-[1-(2-fluorobenzyl)-1 H-pyrazolo[3,4-b]pyridine-3-yl]-5-pyrimidinyl(methyl)carbamate (3) for the manufacture of a medicament for the treatment of Cystic Fibrosis (CF).

4. Use according to claims 1 to 2 of 3-(4-amino-5-cyclopropylpyrimidine-2-yl)-1-(2-fluorobenzyl)1 H-pyrazolo[3,4-b]pyridine (4a) for the manufacture of a medicament for the treatment of Cystic Fibrosis (CF).

5. Pharmaceutical formulation comprising at least one compound according to claims 1 to 4 for the use in patients suffering from Cystic Fibrosis (CF).

6. Combination of at least one sGC stimulator or activator selected from the group comprising 2-[1-(2-fluorobenzyl)-1 H-pyrazolo[3,4-b]pyridine-3-yl]-5-(4-morpholinyl)-4,6-pyrimidinediamine (1), 2-[1-(2-fluorobenzyl)-1 H-pyrazolo[3,4-b]pyridine-3-yl]-5-(4-pyridinyl)-4-pyrimidinamine (2), methyl-4,6-diamino-2-[1-(2-fluorobenzyl)-1 H-pyrazolo[3,4-b]pyridine-3-yl]-5-pyrimidinyl(methyl)carbamate (3), methyl-4,6-diamino-2-[1-(2-fluorobenzyl)-1 H-pyrazolo[3,4-b]pyridine-3-yl]-5-pyrimidinylcarbamate (4), 3-(4-amino-5-cyclopropylpyrimidine-2-yl)-1-(2-fluorobenzyl)1 H-pyrazolo[3,4-b]pyridine (4a), 5-chloro-2-(5-chlorothiophene-2-sulfonylamino-N-(4-(morpholine-4-sulfonyl)-phenyl)-benzamide sodium salt (6), 2-(4-chloro-phenylsulfonylamino)-4,5-dimethoxy-N-(4-(thiomorpholine-4-sulfonyl)-phenyl)-benzamide (7), and/or 4-({(4-carboxybutyl)[2-(2-{[4-(2-phenylethyl) benzyl] oxy}phenyl)ethyl]amino}methyl) benzoic acid (5), 1-{6-[5-chloro-2-({4-trans-4-}trifluoromethyl)cyclohexyl]benzyl}oxy)phenyl] pyridin-2-yl}-5-(trifluoromethyl)-1H-pyrazole-4-carboxylic acid (8), 1-[6-(2-(2-methyl-4-(4-trifluoromethoxyphenyl)benzyloxy)-phenyl)pyridin-2-yl]-5-trifluoromethyl-pyrazole-4-carboxylic acid (9), 1[6-(3,4-dichlorophenyl)-2-pyridinyl-5-(trifluoromethyl)-1H-pyrazole-4-carboxylic acid (10), 1-({2-[3-chloro-5-(trifluoromethyl)phenyl]-5-methyl-1,3-thiazol-4-yl}methyl)-1H-pyrazole-4-carboxylic acid (11), 4-({2-[3-(trifluoromethyl)phenyl]-1,3-thiazol-4-yl}methyl)benzoic acid (12), 1-({2-[2-fluoro-3-(trifluoromethyl)phenyl]-5-methyl-1,3-thiazol-4-yl}methyl)-1H-pyrazole-4-carboxylic acid (13) with a PDE5 inhibitor selected form the group comprising Vardenafil, Sildenafil, Tadalafil, Udenafil, Dasantafil, Avanafil, Mirodenafil, Lodenafil, UK 369.003, UK 371.800, SLx2101 and LAS34179 in which the combination comprises 0.01 to 10 mg of the sGC stimulator or activator and 2.5 to 20 mg of the PDE5 inhibitor.

7. Combination according to claim 6 in which the sGC stimulator is methyl-4,6-diamino-2-[1-(2-fluorobenzyl)-1 H-pyrazolo[3,4-b]pyridine-3-yl]-5-pyrimidinyl-(methyl)carbamate (3) or methyl-4,6-diamino-2-[1-(2-fluorobenzyl)-1 H-pyrazolo[3,4-b]pyridine-3-yl]-5-pyrimidinylcarbamate (4).

8. Combination according to claims 6 and 7 in which the PDE5 inhibitor is Vardenafil or Sildenafil.

9. Combination according to claims 6 to 8 for the use as a medicament.

10. Use of combination according to claims 6 to 9 for the manufacture of a medicament for the treatment of Cystic Fibrosis (CF).

11. Combination according to claims 6 to 9 for the use in patients suffering from Cystic Fibrosis (CF).

12. Pharmaceutical formulation comprising at least one combination according to claims 6 to 8.

## Patentansprüche

1. Verwendung eines sGC-Stimulators oder -Aktivators ausgewählt aus der Gruppe umfassend 2-[1-(2-Fluorbenzyl)-1H-pyrazolo[3,4-b]pyridin-3-yl]-5-(4-morpholinyl)-4,6-pyrimidindiamin (1), 2-[1-(2-Fluorbenzyl)-1H-pyrazolo[3,4-b]pyridin-3-yl]-5-(4-pyridinyl)-4-pyrimidinamin (2), 4,6-Diamino-2-[1-(2-fluorbenzyl)-1H-pyrazolo[3,4-b]pyridin-3-yl]-5-pyrimidinyl(methyl)carbamidsäuremethylester (3), 4,6-Diamino-2-[1-(2-fluorbenzyl)-1H-pyrazolo[3,4-b]pyridin-3-yl]-5-pyrimidinylcarbamidsäuremethyl-ester (4), 3-(4-Amino-5-cyclopropylpyrimidin-2-yl)-1-(2-fluorbenzyl)-1H-pyrazolo[3,4-b]pyridin (4a), 5-Chlor-2-(5-chlorthiophen-2-sulfonylamino-N-(4-(morpholin-4-sulfonyl)phenyl)benzamid-Natriumsalz (6), 2-(4-Chlorphenylsulfonylamino)-4,5-dimethoxy-N-(4-(thiomorpholin-4-sulfonyl)phe-nyl)benzamid (7) und/oder 4-({(4-Carboxybutyl)[2-(2-{[4-(2-phenylethyl)benzyl]oxy}phenyl)ethyl]ami-no}methyl)benzoesäure (5), 1-{6-[5-Chlor-2-({4-trans-4-}trifluormethyl)cyclohexyl]benzyl}oxy)phe-nyl]pyridin-2-yl}-5-(trifluormethyl)-1H-pyrazol-4-carbonsäure (8), 1-[6-(2-(2-Methyl-4-(4-trifluor-methoxyphenyl)benzyloxy)phenyl)pyridin-2-yl]-5-trifluormethylpyrazol-4-carbonsäure (9), 1-[6-(3,4-Dichlorphenyl)-2-pyridinyl-5-(trifluorme-thyl)-1H-pyrazol-4-carbonsäure (10), 1-({2-[3-Chlor-5-(trifluormethyl)phenyl]-5-methyl-1,3-thiazol-4-yl}methyl)-1H-pyrazol-4-carbonsäure (11), 4-({2-[3-(Trifluormethyl)phenyl]-1,3-thiazol-4-yl}methyl)benzoesäure (12), 1-({2-[2-Fluor-3-(trifluormethyl)phenyl]-5-methyl-1,3-thiazol-4-yl}methyl)-1H-pyrazol-4-carbonsäure (13) zur Herstellung eines Medikaments zur Behandlung von zystischer Fibrose (ZF).

2. Verwendung nach Anspruch 1 eines sGC-Stimulators ausgewählt aus der Gruppe umfassend 4,6-Diamino-2-[1-(2-fluorbenzyl)-1H-pyrazolo[3,4-b]pyridin-3-yl]-5-pyrimidinyl(methyl)carbamidsäuremethylester (3), 4,6-Diamino-2-[1-(2-fluorbenzyl)-1H-pyra-zolo[3,4-b]pyridin-3-yl]-5-pyrimidinylcarbamid-säuremethylester (4), 3-(4-Amino-5-cyclopropylpy-rimidin-2-yl)-1-(2-fluorbenzyl)-1H-pyrazolo[3,4-b]pyridin (4a) zur Herstellung eines Medikaments zur Behandlung von zystischer Fibrose (ZF).

3. Verwendung nach Anspruch 1 oder 2 von 4,6-Diamino-2-[1-(2-fluorbenzyl)-1H-pyrazolo[3,4-b]pyridin-3-yl]-5-pyrimidinyl(methyl)carbamidsäuremethylester (3) zur Herstellung eines Medikaments zur Behandlung von zystischer Fibrose (ZF).

4. Verwendung nach Anspruch 1 oder 2 von 3- (4-Amino-5-cyclopropylpyrimidin-2-yl)-1-(2-fluorbenzyl)-1H-pyrazolo[3,4-b]pyridin (4a) zur Herstellung eines Medikaments zur Behandlung von zystischer Fibrose (ZF).

5. Pharmazeutische Formulierung, umfassend mindestens eine Verbindung nach einem der Ansprüche 1 bis 4 zur Verwendung in an zystischer Fibrose (ZF) leidenden Patienten.

6. Kombination mindestens eines sGC-Stimulators oder -Aktivators ausgewählt aus der Gruppe umfassend 2-[1-(2-Fluorbenzyl)-1H-pyrazolo[3,4-b]pyridin-3-yl]-5-(4-morpholinyl)-4,6-pyrimidindiamin (1), 2-[1-(2-Fluorbenzyl)-1H-pyrazolo[3,4-b]pyridin-3-yl]-5-(4-pyridinyl)-4-pyrimidinamin (2), 4,6-Diamino-2-[1-(2-fluorbenzyl)-1H-pyrazolo[3,4-b]pyridin-3-yl]-5-pyrimidinyl(methyl)carbamidsäuremethylester (3), 4,6-Diamino-2-[1-(2-fluorbenzyl)-1H-pyrazolo[3,4-b]pyridin-3-yl]-5-pyrimidinylcarbamidsäuremethyl-ester (4), 3-(4-Amino-5-cyclopropylpyrimidin-2-yl)-1-(2-fluorbenzyl)-1H-pyrazolo[3,4-b]pyridin (4a), 5-Chlor-2-(5-chlorthiophen-2-sulfonylamino-N-(4-(morpholin-4-sulfonyl)phenyl)benzamid-Natriumsalz (6), 2-(4-Chlorphenylsulfonylamino)-4,5-dimethoxy-N-(4-(thiomorpholin-4-sulfonyl)phe-nyl)benzamid (7) und/oder 4-({(4-Carboxybutyl)[2-(2-{[4-(2-phenylethyl)benzyl]oxy}phenyl)ethyl]amino}methyl)benzoesäure (5), 1-{6-[5-Chlor-2-({4-trans-4-}trifluormethyl)cyclohexyl]benzyl}oxy)phenyl]pyridin-2-yl}-5-(trifluormethyl)-1H-pyrazol-4-carbonsäure (8), 1-[6-(2-(2-Methyl-4-(4-trifluor-methoxyphenyl)benzyloxy)phenyl)pyridin-2-yl]-5-trifluormethylpyrazol-4-carbonsäure (9), 1-[6-(3,4-Dichlorphenyl)-2-pyridinyl-5-(trifluorme-thyl)-1H-pyrazol-4-carbonsäure (10), 1-({2-[3-Chlor-5-(trifluormethyl)phenyl]-5-methyl-1,3-thiazol-4-yl}methyl)-1H-pyrazol-4-carbonsäure (11), 4-({2-[3-(Trifluormethyl)phenyl]-1,3-thiazol-4-yl}methyl)benzoesäure (12), 1-({2-[2-Fluor-3-(trifluormethyl)phenyl]-5-methyl-1,3-thiazol-4-yl}methyl)-1H-pyrazol-4-carbonsäure (13) mit einem PDE5-Inhibitor ausgewählt aus der Gruppe umfassend Vardenafil, Sildenafil, Tadalafil, Udenafil, Dasantafil, Avanafil, Mirodenafil, Lodenafil, UK 369.003, UK 371.800, SLx2101 und LAS34179, wobei die Kombination 0,01 bis 10 mg des sGC-Stimulators bzw. -Aktivators und 2,5 bis 20 mg des PDE5-Inhibitors umfasst.

7. Kombination nach Anspruch 6, wobei es sich bei dem sGC-Stimulator um 4,6-Diamino-2-[1-(2-fluorbenzyl)-1H-pyrazolo[3,4-b]pyridin-3-yl]-5-pyrimidinyl(methyl)carbamidsäuremethylester (3) oder 4,6-Diamino-2-[1-(2-fluorbenzyl)-1H-pyrazo-lo[3,4-b]pyridin-3-yl]-5-pyrimidinylcarbamidsäure-methylester (4) handelt.

8. Kombination nach Anspruch 6 oder 7, wobei es sich bei dem PDE5-Inhibitor um Vardenafil oder Sildenafil handelt.

9. Kombination nach einem der Ansprüche 6 bis 8 zur Verwendung als Medikament.

10. Verwendung einer Kombination nach einem der Ansprüche 6 bis 9 zur Herstellung eines Medikaments zur Behandlung von zystischer Fibrose (ZF).

11. Kombination nach einem der Ansprüche 6 bis 9 zur Verwendung in an zystischer Fibrose (ZF) leidenden Patienten.

12. Pharmazeutische Formulierung, umfassend mindestens eine Kombination nach einem der Ansprüche 6 bis 8.

## Revendications

1. Utilisation d'un agent activateur ou stimulant de sGC choisi dans le groupe comprenant les suivants : 2-[1-(2-fluorobenzyl)-1H-pyrazolo[3,4-b]pyridine-3-yl]-5-(4-morpholinyl)-4,6-pyrimidine-diamine (1), 2-[1-(2-fluorobenzyl)-1H-pyrazolo[3,4-b]pyridine-3-yl]-5-(4-pyridinyl)-4-pyrimidinamine (2), carbamate de méthyl-4,6-diamino-2-[1-(2-fluorobenzyl)-1H-pyrazolo[3,4-b]pyridine-3-yl]-5-pyrimidinyl(méthyle) (3), carbamate de méthyl-4,6-diamino-2-[1-(2-fluorobenzyl)-1H-pyrazolo[3,4-b]pyridine-3-yl]-5-pyrimidinyle (4), 3-(4-amino-5-cyclopropylpyrimidine-2-yl)-1-(2-fluorobenzyl)1H-pyrazolo[3,4-b]pyridine (4a), 5-chloro-2-(5-chlorothiophène-2-sulfonylamino-N-(4-(morpholine-4-sulfonyl)-phényl)-benzamide, sel de sodium (6), 2-(4-chloro-phénylsulfonylamino)-4,5-diméthoxy-N-(4-(thiomorpholine-4-sulfonyl)-phényl)-benzamide (7), et/ou acide 4-({(4-carboxybutyl)[2-(2-{[4-(2-phényléthyl)benzyl]oxy}phényl)éthyl]amino}méthyl)benzoï que (5), acide 1-{6-[5-chloro-2-({4-trans-4-}trifluorométhyl)cyclohexyl]benzyl}oxy)phényl]pyridin-2-yl}-5-(trifluorométhyl)-1H-pyrazole-4-carboxylique (8), acide 1-[6-(2-(2-méthyl-4-(4-trifluorométhoxyphényl)benzyloxy)-phényl)pyridin-2-yl]-5-trifluorométhyl-pyrazole-4-carboxylique (9), acide 1-[6-(3,4-dichlorophényl)-2-pyridinyl-5-(trifluorométhyl)-1H-pyrazole-4-carboxylique (10), acide 1-({2-[3-chloro-5-(trifluorométhyl)phényl]-5-méthyl-1,3-thiazol-4-yl}méthyl)-1H-pyrazole-4-carboxylique (11), acide 4-({2-[3-(trifluorométhyl)phényl]-1,3-thiazol-4-yl}méthyl)benzoïque (12), acide 1-({2-[2-fluoro-3-(trifluorométhyl)phényl]-5-méthyl-1,3-thiazol-4-yl}méthyl)-1H-pyrazole-4-carboxylique (13) dans la fabrication d'un médicament destiné au traitement de la mucoviscidose (MV).

2. Utilisation selon la revendication 1 d'un agent stimulant de sGC choisi dans le groupe comprenant les suivants : carbamate de méthyl-4,6-diamino-2-[1-(2-fluorobenzyl)-1H-pyrazolo[3,4-b]pyridine-3-yl]-5-pyrimidinyl(méthyle) (3), carbamate de méthyl-4,6-diamino-2-[1-(2-fluorobenzyl)-1H-pyrazolo[3,4-b]pyridine-3-yl]-5-pyrimidinyle (4), 3-(4-amino-5-cyclopropylpyrimidine-2-yl)-1-(2-fluorobenzyl)1H-pyrazolo[3,4-b]pyridine (4a) dans la fabrication d'un médicament destiné au traitement de la mucoviscidose (MV).

3. Utilisation selon les revendications 1 et 2 du carbamate de méthyl-4,6-diamino-2-[1-(2-fluorobenzyl)-1H-pyrazolo[3,4-b]pyridine-3-yl]-5-pyrimidinyl(méthyle) (3) dans la fabrication d'un médicament destiné au traitement de la mucoviscidose (MV).

4. Utilisation selon les revendications 1 à 2 de la 3-(4-amino-5-cyclopropylpyrimidine-2-yl)-1-(2-fluorobenzyl)-1H-pyrazolo[3,4-b]pyridine (4a) dans la fabrication d'un médicament destiné au traitement de la mucoviscidose (MV).

5. Formule pharmaceutique comprenant au moins un composé selon les revendications 1 à 4 pour utilisation chez des patients souffrant de mucoviscidose (MV).

6. Combinaison d'au moins un agent activateur ou stimulant de sGC choisi dans le groupe comprenant les suivants : 2-[1-(2-fluorobenzyl)-1H-pyrazolo[3,4-b]pyridine-3-yl]-5-(4-morpholinyl)-4,6-pyrimidine-diamine (1), 2-[1-(2-fluorobenzyl)-1H-pyrazolo[3,4-b]pyridine-3-yl]-5-(4-pyridinyl)-4-pyrimidinamine (2), carbamate de méthyl-4,6-diamino-2-[1-(2-fluorobenzyl)-1H-pyrazolo[3,4-b]pyridine-3-yl]-5-pyrimidinyl(méthyle) (3), carbamate de méthyl-4,6-diamino-2-[1-(2-fluorobenzyl)-1H-pyrazolo[3,4-b]pyridine-3-yl]-5-pyrimidinyle (4), 3-(4-amino-5-cyclopropylpyrimidine-2-yl)-1-(2-fluorobenzyl)1H-pyrazolo[3,4-b]pyridine (4a), 5-chloro-2-(5-chlorothiophène-2-sulfonylamino-N-(4-(morpholine-4-sulfonyl)-phényl)-benzamide, sel de sodium (6), 2-(4-chloro-phénylsulfonylamino)-4,5-diméthoxy-N-(4-(thiomorpholine-4-sulfonyl)-phényl)-benzamide (7), et/ou acide 4-({(4-carboxybutyl)[2-(2-{[4-(2-phényléthyl)benzyl]oxy}phényl)éthyl]amino}méthyl)benzoï que (5), acide 1-{6-[5-chloro-2-({4-trans-4-}trifluorométhyl)cyclohexyl]benzyl}oxy)phényl]pyridin-2-yl}-5-(trifluorométhyl)-1H-pyrazole-4-carboxylique (8), acide 1-[6-(2-(2-méthyl-4-(4-trifluorométhoxyphényl)benzyloxy)-phényl)pyridin-2-yl]-5-trifluorométhyl-pyrazole-4-carboxylique (9), acide 1-[6-(3,4-dichlorophényl)-2-pyridinyl-5-(trifluorométhyl)-1H-pyrazole-4-carboxylique (10), acide 1-({2-[3-chloro-5-(trifluorométhyl)phényl]-5-méthyl-1,3-thiazol-4-yl}méthyl)-1H-pyrazole-4-carboxylique (11), acide 4-({2-[3-(trifluorométhyl)phényl]-1,3-thiazol-4-yl}méthyl)benzoïque (12), acide 1-({2-[2-fluoro-3-(trifluorométhyl)phényl]-5-méthyl-1,3-thiazol-4-yl}méthyl)-1H-pyrazole-4-carboxylique (13) avec un inhibiteur de PDE5 choisi dans le groupe comprenant les suivants : Vardénafil, Sildénafil, Tadalafil, Udénafil, Dasantafil, Avanafil, Mirodénafil, Lodénafil, UK 369.003, UK 371.800, SLx2101 et LAS34179 dans lequel la combinaison comprend 0,01 à 10 mg de l'agent activateur ou stimulant de sGC et 2,5 à 20 mg de l'inhibiteur de PDE5.

7. Combinaison selon la revendication 6 dans lequel l'agent stimulant de sGC est le carbamate de méthyl-4,6-diamino-2-[1-(2-fluorobenzyl)-1H-pyrazolo[3,4-b]pyridine-3-yl]-5-pyrimidinyl-(méthyle) (3) ou le carbamate de méthyl-4,6-diamino-2-[1-(2-fluorobenzyl)-1H-pyrazolo[3,4-b]pyridine-3-yl]-5-pyrimidinyle (4).

8. Combinaison selon les revendications 6 et 7 dans lequel l'inhibiteur de PDE5 est le Vardénafil ou le Sildénafil.

9. Combinaison selon les revendications 6 à 8 pour utilisation en tant que médicament.

10. Utilisation d'une combinaison selon les revendications 6 à 9 dans la fabrication d'un médicament destiné au traitement de la mucoviscidose (MV).

11. Combinaison selon les revendications 6 à 9 pour utilisation chez des patients souffrant de mucoviscidose (MV).

12. Formule pharmaceutique comprenant au moins une combinaison selon les revendications 6 à 8.
